# FASCICULE DE BREVET EUROPEEN

(11) **EP 2 424 878 B1**
(45) Date de publication et mention de la délivrance du brevet: **06.03.2013**
(21) Numéro de dépôt: 10727044.9
(22) Date de dépôt: 27.04.2010
(51) Int. Cl.: C12N 15/10, C07K 14/005, C12N 11/16

(54) **MODIFICATION DU GENOME D'UN BACTERIOPHAGE LYTIQUE PAR IMMOBILISATION DUDIT BACTERIOPHAGE DANS SA BACTERIE HOTE**
MODIFIKATION DES GENOMS EINES LYTISCHEN BAKTERIOPHAGEN DURCH IMMOBILISIEREN DES BAKTERIOPHAGEN IN DESSEN WIRTSBAKTERIUM
MODIFICATION OF THE GENOME OF A LYTIC BACTERIOPHAGE BY IMMOBILIZING SAID BACTERIOPHAGE IN THE HOST BACTERIUM THEREOF

(30) Priorité: 30.04.2009 FR 0952933
(43) Date de publication de la demande: 07.03.2012
(73) Titulaire: Pherecydes Pharma, 93230 Romainville (FR)
(72) Inventeur: POUILLOT, Flavie, F-75011 Paris (FR); IRIS, François, F-92370 Chaville (FR)
(74) Mandataire: Santarelli
(86) Numéro de dépôt international: PCT/FR2010/050796
(87) Numéro de publication internationale: WO 2010/125296

(56) Documents cités:
- WO-A2-2008/093009
- XU YI ET AL: "Mutations in the Rho transcription termination factor that affect RNA tracking" JOURNAL OF BIOLOGICAL CHEMISTRY, vol. 277, no. 33, 16 août 2002 (2002-08-16), pages 30023-30030, XP002562011 ISSN: 0021-9258
- MIWA YOSHIHIRO ET AL: "Structural and functional dissections of transcription termination factor Rho by random mutagenesis" JOURNAL OF MOLECULAR BIOLOGY, LONDON, GB, vol. 254, no. 5, 1 janvier 1995 (1995-01-01), pages 815-837, XP002241067 ISSN: 0022-2836
- MARTINEZ A ET AL: "Mutational analysis and secondary structure model of the RNP1-like sequence motif of transcription termination factor rho" JOURNAL OF MOLECULAR BIOLOGY, LONDON, GB, vol. 257, no. 5, 1 janvier 1996 (1996-01-01), pages 895-908, XP002353473 ISSN: 0022-2836
- YOICHI M ET AL: "Alteration of tail fiber protein gp38 enables T2 phage to infect Escherichia coli O157:H7" JOURNAL OF BIOTECHNOLOGY, ELSEVIER SCIENCE PUBLISHERS, AMSTERDAM, NL, vol. 115, no. 1, 12 janvier 2005 (2005-01-12), pages 101-107, XP004966993 ISSN: 0168-1656
- CASTILLA-LLORENTE VIRGINIA ET AL: "kinC/D-mediated heterogeneous expression of spo0A during logarithmical growth in Bacillus subtilis is responsible for partial suppression of phi 29 development" MOLECULAR MICROBIOLOGY, vol. 68, no. 6, juin 2008 (2008-06), pages 1406-1417, XP002562013 ISSN: 0950-382X
- POUILLOT FLAVIE ET AL: "Genetically engineered virulent phage banks in the detection and control of emergent pathogenic bacteria." BIOSECURITY AND BIOTERRORISM : BIODEFENSE STRATEGY, PRACTICE, AND SCIENCE JUN 2010 LNKD- PUBMED:20569057, vol. 8, no. 2, juin 2010 (2010-06), pages 155-169, XP002594997 ISSN: 1557-850X

## Description

La présente invention concerne un procédé permettant l'immobilisation réversible de bactériophages lytiques à l'intérieur de leurs hôtes bactériens modifiés.

Elle a plus particulièrement pour objet un procédé pour modifier le génome d'un bactériophage lytique grâce à l'immobilisation dudit bactériophage dans sa bactérie hôte.

### PREAMBULE

Les bactériophages se présentent comme des virus ayant une taille comprise entre environ 24 à 200 nm, capables d'infecter spécifiquement les bactéries.

Les bactériophages, dans leur forme libre, sont constitués d'une enveloppe protéique externe, appelée capside, renfermant le matériel génétique, qui constitue leur génome. Le génome de la grande majorité des bactériophages est constitué d'une molécule d'ADN double-brin linéaire d'une taille comprise entre environ 5 et 650 kb. La capside est généralement prolongée par une queue servant à injecter ledit matériel génétique dans la bactérie hôte.

Il existe une grande diversité de bactériophages dans le milieu naturel.

Toutefois, les bactériophages sont généralement très spécifiques et ne peuvent infecter qu'un très petit nombre d'espèces bactériennes. Cette spécificité étroite est liée, en particulier, à leur mécanisme de pénétration dans la cellule bactérienne : les phages sont capables de reconnaître spécifiquement les structures glycoprotéiques se trouvant à la surface de la paroi de leur bactérie hôte, qui leur servent de "récepteurs". Ces structures leur permettent de s'accrocher à la paroi et d'injecter leur matériel génétique à l'intérieur du cytoplasme de leur bactérie hôte. Cette reconnaissance s'opère par des protéines dites de « ciblage », qui sont présentes à la surface de la capside où à l'extrémité des filaments du bactériophage, appelés également « fibres de queue ».

Les bactériophages sont classés en fonction de leurs cycles de réplication. On distingue ainsi trois grandes catégories de bactériophages :

Une première catégorie de bactériophages sont dits « lytiques » ou « virulents », c'est-à-dire qu'aussitôt qu'ils infectent une bactérie, l'expression et la réplication de leur génome donnent lieu à l'assemblage de nouvelles particules de phages, et, dans un court laps de temps, provoquent la lyse de la bactérie hôte et la libération d'une descendance multiple.

Le bactériophage T4, par exemple, est un bactériophage qui infecte la bactérie *Escherichia coli.* Son cycle lytique dure environ 30 minutes à 37 °C. Ce cycle débute immédiatement après la reconnaissance de la bactérie hôte par le bactériophage par une phase d'absorption et de pénétration. Il se traduit par l'arrêt immédiat de l'expression des gènes de la bactérie hôte au profit de ceux du bactériophage permettant la synthèse des enzymes nécessaires à la réplication du bactériophage. Puis, environ 10 minutes après l'infection, s'effectuent la réplication de l'ADN et la synthèse des protéines virales et l'assemblage des bactériophages fils (démarrant après 12 minutes). Le cycle de réplication conduit à l'éclatement de la bactérie (après 30 minutes) et la libération dans l'environnement d'environ cinquante bactériophages par bactérie lysée.

Une seconde catégorie de bactériophages sont dits « lysogéniques » ou « tempérés ». Ces bactériophages peuvent demeurer dans un état quiescent en intégrant leur matériel génétique à celui de la bactérie. On parle alors de provirus ou de prophage, c'est à dire un virus dont le matériel génétique est intégré au chromosome de la bactérie hôte. Le génome du bactériophage est copié à chaque division cellulaire avec l'ensemble de l'ADN de la bactérie, que l'on qualifie alors de lysogène. Pendant cette phase de latence, l'expression des gènes codés par le génome du phage est en général réprimée par une protéine répresseur. Dans certaines conditions, en particulier en cas de carence ou de stress, le prophage sort de son état quiescent et active son cycle réplicatif. Il s'excise du génome de l'hôte et entre dans un cycle lytique, tel que décrit ci-dessus.

Une troisième catégorie de bactériophages ne provoquent pas la lyse de la cellule infectée, mais bourgeonnent à la surface de la membrane bactérienne, sans la rompre. C'est le cas des bactériophages filamenteux de type M13 ou f1 d'*Escherichia coli,* qui sont utilisés dans les techniques bien connues de « phage display ». La bactérie infectée survit à l'infection et produit des bactériophages de manière continue.

YOICHI M et al. ("Alteration of tail fiber protein gp38 enables T2 phage to infect Escherichia coli O157:H7" JOURNAL OF BIOTECHNOLOGY vol. 115, no. 1, 12 janvier 2005, pages 101-107) décrit un procédé pour modifier le génome d'un bactériophage lytique (T2) dans une bactérie hôte (E.coli K12).

XU YI et al. ("Mutations in the Rho transcription termination factor that affect RNA tracking" JOURNAL OF BIOLOGICAL CHEMISTRY, vol. 277, no. 33, 16 août 2002, pages 30023-30030) décrit quatre systèmes d'expression de protéines Rho mutées, chacun de ces systèmes comprenant une souche bactérienne hôte dont la copie chromosomique de rho est sauvage et un vecteur comprenant une copie mutée.

MIWA YOSHIHIRO et al. ("Structural and functional dissections of transcription termination factor Rho by random mutagenesis" JOURNAL OF MOLECULAR BIOLOGY vol. 254, no. 5, 1 janvier 1995, pages 815-837) décrit la mutation aléatoire de Rho, le clonage dans un vecteur plasmidique d'expression (pEC22) et la transformation dans un hôte sauvage pour rho (E. coli XL1 Blue).

MARTINEZ A et al. ("Mutational analysis and secondary structure model of the RNP1-like séquence motif of transcription termination factor rho" JOURNAL OF MOLECULAR BIOLOGY vol. 257, no. 5, 1 janvier 1996, pages 895-908) décrit une bactérie hôte E. coli AMO13 comprenant une délétion de la copie chromosomique de rho, un vecteur pPMrhoCam comportant une copie sauvage de rho, et un vecteur p93 contenant une copie rho15ts.

La présente invention concerne la première catégorie des bactériophages dits « lytiques » évoquée ci-dessus.

Les bactériophages lytiques ont la particularité de tuer les bactéries qu'ils infectent, ce qui rend possible leur utilisation en tant qu'agents antibactériens.

L'idée d'utiliser des préparations de bactériophages lytiques pour lutter contre des infections bactériennes est ancienne et remonte à l'époque où les antibiotiques n'avaient pas encore été découverts.

L'efficacité des bactériophages pour lutter contre des infections bactériennes a été démontrée. Cependant, leur utilisation est restée très marginale par rapport à celle des antibiotiques. Le spectre d'activité des bactériophages est en effet beaucoup plus étroit que celui des antibiotiques, ce qui limite considérablement leur intérêt, notamment dans le domaine des traitements préventifs.

Aujourd'hui, face à l'apparition de souches bactériennes multi-résistantes aux antibiotiques et aux difficultés rencontrées par la communauté scientifique pour mettre au point de nouveaux antibiotiques, les bactériophages connaissent toutefois un regain d'intérêt, notamment en vue de lutter contre les contaminations nosocomiales [Thiel, K., Nature Biotechnology, 2004, 22:31-36], c'est-à-dire à l'encontre de bactéries ciblées, multi-résistantes ou émergentes.

Dans une précédente demande WO 2008/093009, les présents inventeurs ont décrit un procédé permettant d'obtenir des bactériophages recombinants dont les protéines de ciblage ont été modifiées au hasard. Cette modification s'effectue par l'insertion au niveau des gènes codant les protéines de ciblage de séquences oligonucléotidiques produites de manière aléatoire, de telle sorte que les bactériophages obtenus acquièrent la capacité de reconnaître et d'infecter de nouvelles bactéries, de préférence différentes de leur hôte habituel.

L'obtention de tels bactériophages, ayant un spectre élargi ou différent de celui de leur bactérie hôte, représentait un défi, car lorsque les protéines de ciblage d'un bactériophage sont modifiées, celui-ci peut perdre la faculté de reconnaître son hôte. Il en résulte qu'il ne peut être reproduit, ni maintenu dans son hôte.

Pour surmonter cette difficulté, les inventeurs ont proposé, dans leur précédente invention, de créer une banque de bactéries hôtes comprenant une pluralité de vecteurs de recombinaison homologues, à l'intérieur desquels ont été clonées des séquences nucléotidiques produites de manière aléatoire. Ces bactéries ont été infectées en masse par des bactériophages, de telle sorte à ce que le plus grand nombre possible de séquences aléatoires contenues dans ces vecteurs viennent s'insérer par recombinaison homologue dans les gènes codant les protéines de ciblage. Les bactériophages recombinants ainsi modifiés ont été récupérés après un seul cycle de réplication lytique pour former une banque de bactériophages diversifiés. Une telle banque de bactériophages est alors conservée, en vue d'être utilisée à l'encontre de bactéries cibles que l'on cherche à éliminer ou contrôler.

Toutefois, cette invention, ainsi que la plupart des procédés de modification des bactériophages lytiques, connaissent un point de limitation dans le fait que la réplication des bactériophages s'effectue dans un court laps de temps, qui correspond à celle de la durée du cycle lytique.

Or, pour pouvoir préserver la diversité des bactériophages modifiés, plus particulièrement ceux dont les protéines de ciblage ont été modifiées, il est important de récupérer les bactériophages de première génération à l'issu du premier cycle d'infection. Si les bactériophages infectent une nouvelle fois leur hôte, leur diversité s'en retrouve diminuée du fait que, seuls les bactériophages reconnaissant leur hôte, sont reproduits.

Cette limitation temporelle, liée à la durée du cycle lytique, réduit la fréquence des événements de recombinaisons génétiques, et donc, limite la diversité des bactériophages modifiés pouvant en résulter.

Une autre limitation, indépendante de la première, réside dans le fait que la banque de bactériophages modifiés obtenue, ne peut être reproduite. Il est donc nécessaire, dès lors qu'on cherche à produire un nouveau stock de bactériophages modifiés, d'infecter de nouveau un stock de bactéries hôtes disposant d'une pluralité de vecteurs permettant l'insertion de séquences aléatoires.

La présente invention vise à remédier à ces limitations, et plus particulièrement à s'affranchir des contraintes liées au cycle lytique imposées par ce type de bactériophages.

Les inventeurs ont constaté, de manière surprenante, qu'en surexprimant une forme modifiée de la protéine Rho dans une bactérie infectée par un bactériophage, il était possible d'inhiber le cycle lytique du phage chez cette bactérie et d'empêcher, au moins temporairement, la production d'une progéniture du bactériophage et la lyse des bactéries.

Du fait de cette inhibition, les inventeurs ont mis au point un procédé permettant d'immobiliser un bactériophage lytique dès son entrée dans une bactérie hôte.

Par immobilisation, il faut entendre que le bactériophage, ou plutôt son génome, est retenu à l'intérieur de la bactérie hôte de manière contrôlée.

A leur connaissance, c'est la première fois qu'un phage lytique est ainsi immobilisé, de manière inductible, à l'intérieur de sa bactérie hôte.

Il convient d'avoir à l'esprit que la réplication du génome des bactériophages est un processus très complexe, dont on pense qu'il est indépendant de la duplication des chromosomes bactériens. Cette réplication fait intervenir à la fois des étapes de transcription, de traduction et de duplication de l'ADN.

L'inhibition du cycle lytique selon l'invention, ne semble pas affecter de manière significative le processus de duplication de l'ADN, notamment du génome bactérien. Ainsi, les inventeurs ont établi qu'il était possible de modifier, de manière très efficace, le génome du bactériophage dans la bactérie au cours de sa période d'immobilisation.

En outre, pendant la période dite d'immobilisation, l'intégrité de la bactérie est conservée, de telle sorte qu'elle peut être transformée, notamment par électroporation. De ce fait, le génome du bactériophage peut être modifié à l'intérieur de la bactérie, en particulier par recombinaison homologue, en faisant intervenir du matériel génétique exogène tel que des fragments de PCR ou des vecteurs.

Les formes modifiées de la protéine Rho, qui ont été utilisées par les inventeurs pour parvenir à l'immobilisation des bactériophages selon l'invention, résultent plus particulièrement de l'expression de formes mutées de gènes rho.

Les gènes rho sont présents dans le génome de très nombreuses espèces bactériennes. Il suffit donc, selon l'invention, d'obtenir une copie mutée du gène sauvage présent dans une espèce bactérienne donnée, pour pouvoir obtenir l'immobilisation des bactériophages spécifiques à cette espèce donnée et, le cas échéant, procéder à la modification de son génome.

Le procédé de l'invention, ainsi que les bactéries hôtes mises au point par les inventeurs constituent donc de nouveaux outils de biologie moléculaire permettant d'immobiliser et de modifier le génome des bactériophages lytiques dans un très grand nombre d'hôtes bactériens.

L'invention présente de nombreux avantages détaillés ci-après, lesquels résident notamment en ce que:
- le bactériophage est immobilisé de façon efficace, stable et entièrement contrôlée, et ce par des moyens simples qui peuvent être appliqués dans tout hôte bactérien potentiel ;
- l'immobilisation du bactériophage est réversible et n'affecte ni son caractère lytique, ni son pouvoir de réplication une fois l'immobilisation levée ;
- le mécanisme d'immobilisation résiste à l'électroporation et permet de multiples étapes successives de recombinaisons sur le bactériophage sans affecter l'hôte de manière adverse et sans avoir à recourir à des cycles lytiques successifs, qui, au gré des infections successives, conduisent par sélection vis-à-vis de l'hôte, à une perte de diversité des bactériophages ;
- le bactériophage immobilisé dans l'hôte peut agir comme « vecteur de transduction modulable », c'est-à-dire qu'il peut participer à la transformation de son hôte ou être inséré dans le génome de son hôte, à la manière d'un bactériophage lysogénique.
- la bactérie hôte infectée a la particularité de devenir résistante à toute infection subséquente par un autre bactériophage, ce qui limite les transferts horizontaux d'ADN et augmente la sûreté du procédé de modification du génome.

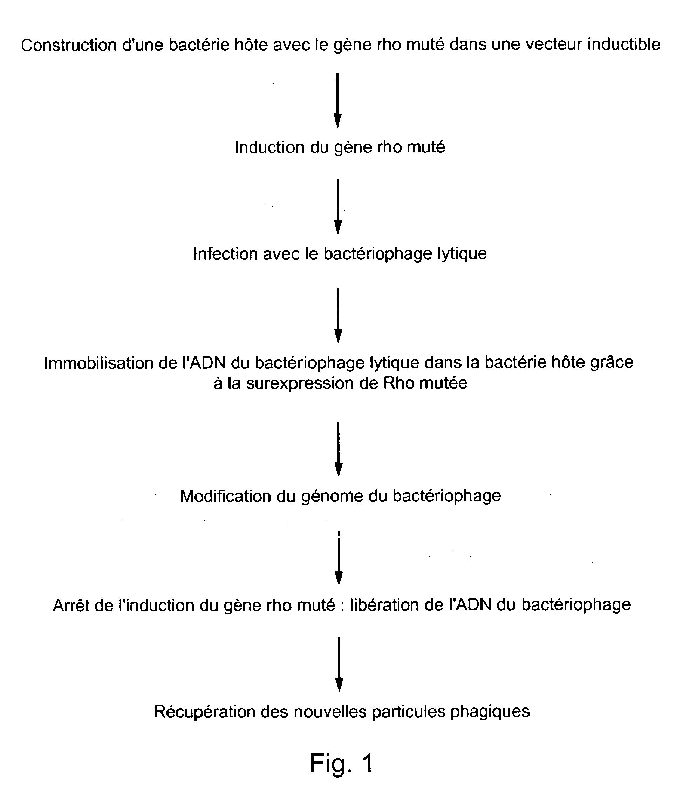

**Figure 1**: Schéma récapitulatif des étapes du procédé selon un mode préféré de l'invention.

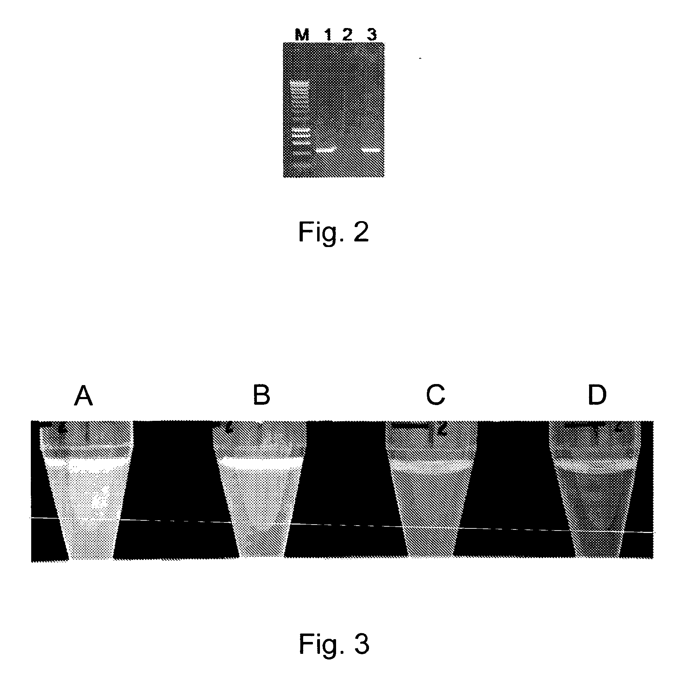

**Figure 2** : Vérification sur gel d'agarose de la présence du génome ADN du bactériophage T4 dans son hôte bactérien E.coli DK8. L'ADN est ici extrait des bactéries après infection de celles-ci par le bactériophage T4 et induction de l'expression de Rho* dans la bactérie. M : marqueurs de tailles moléculaires SmartLadder (Eurogentec). **1** : ADN témoin du bactériophage T4. **2** : Extraction de cellules non infectées d*'E.coli* (témoin négatif). **3** : Extraction du génome du bactériophage T4 des cellules d'*E.coli*, 24 heures après l'induction de Rho*. L'ADN du bactériophage est détecté dans les puits 1 et 3.
**Figure 3** : Suspensions de cultures bactériennes d'*E.coli* infectées par le bactériophage T4. **A**: aspect trouble de la suspension de cellules (non lysées). **B** et **C** : aspect de plus en plus transparent de la suspension du fait de la lyse des cellules suite à la levée de l'immobilisation et la libération de la progéniture du bactériophage. **D** : aspect transparent de la suspension du fait de la lyse totale des cellules en l'absence d'induction de l'expression du gène Rho*. Le phage immobilisé libéré reste infectieux.

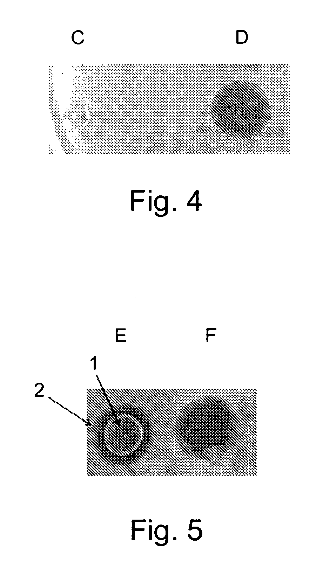

**Figure 4**: Dépôt sur tapis bactérien (en double couche sur milieu nutritif gélosé). Les plages de lyse, plus sombres, révèlent la présence de bactériophages au stade lytique. **C** : Les bactériophages sont en phase d'immobilisation (absence de plage de lyse). **D** : La phase d'immobilisation est levée. Les bactériophages reprennent alors leur cycle lytique (lyse des bactéries visible sur le tapis bactérien).
**Figure 5** : Dépôt sur tapis bactérien (en double couche sur milieu nutritif gélosé). Les plages de lyse, plus sombres, révèlent la présence de bactériophages au stade lytique. **E** : (**1**) bactéries dans lesquels les bactériophages sont immobilisés (**2**) bactériophages libérés après modification de leur génome. **F** : Libération des bactériophages après modification.

### DESCRIPTION DETAILLEE DE L'INVENTION

L'invention concerne dans son principe général un procédé permettant d'immobiliser et de modifier le génome d'un bactériophage lytique dans une bactérie hôte. Ce procédé est caractérisé, notamment, en ce que :
(i) on infecte une bactérie hôte avec un bactériophage ;
(ii) on inhibe le cycle lytique du bactériophage dans son hôte de manière temporaire;
(iii) on modifie le génome du bactériophage pendant l'étape ii) durant laquelle le bactériophage est immobilisé dans la bactérie hôte ;
(iv) on lève l'inhibition du cycle lytique induite à l'étape ii), laquelle peut être qualifiée de temporaire, de sorte à libérer la progéniture du bactériophage de l'étape (i) dont le génome a été modifié. La progéniture, qui constitue la descendance du bactériophage, est constituée de bactériophages fils dont une partie comprend un génome modifié par rapport à celui du bactériophage père, qui a servi à infecter la bactérie hôte.

Par modification du génome d'un bactériophage, on entend tout remaniement du matériel génétique initial d'un bactériophage, quelle que soit la technique utilisée pour y parvenir. Il peut s'agir de mutations ponctuelles, d'insertions ou de délétions. La présente invention facilite plus particulièrement les modifications du génome impliquant les techniques de recombinaison homologue. Les techniques de recombinaison homologue utilisées pour la mise en oeuvre de la présente invention sont connues de l'homme du métier [Poteete, A.R. et al., (2001) FEMS Microbiol. Lett. 201(1):9-14 ; Kuzminov, A. et al. (2001) PNAS, 98(15):8298-305].

De préférence, le bactériophage dont on modifie le génome selon l'invention est un bactériophage de la famille des Myoviridae, préférentiellement de type-T, tels que les bactériophages T4, T5, T6 et T7. Ces bactériophages sont bien connus de l'homme du métier, en particulier le bactériophage T4, dont le génome a été entièrement séquencé [Miller, E.S. et al., Bacteriophage T4 genome, Microbiol Mol. Biol. Rev., 2003, 67(1):86-156]. La séquence complète du génome du bactériophage est disponible dans Genbank (AF 158101).

Une bactérie hôte selon l'invention est une bactérie utilisée couramment pour répliquer le bactériophage dont on cherche à modifier le génome. De préférence, la bactérie hôte est une souche que l'on peut transformer à l'aide d'une construction d'ADN selon l'invention, permettant de modifier le bactériophage par recombinaison homologue.

Une bactérie hôte particulièrement adaptée aux bactériophages de type-T pour la mise en oeuvre du procédé de la présente invention, est une bactérie à gram négatif, plus particulièrement *Escherichia coli.* La souche DK8 (ATCC 47038) est particulièrement adaptée pour modifier le bactériophage T4.

Dans le cadre de la modification du génome des bactériophages de type-T, il peut être avantageux d'utiliser une souche d'*E.coli* transformée à l'aide d'un vecteur de type Mini-lambda (λ), dérivé du prophage lambda et comprenant les gènes *exo, bet* et *gam.* Un tel vecteur permet de contrôler, par exemple, la recombinaison homologue, en fonction de la température à laquelle la bactérie hôte est cultivée, et ainsi mieux contrôler les événements de recombinaison opérés dans la bactérie hôte.

Par « inhibition du cycle lytique », on entend un allongement de la durée du cycle lytique par rapport à la durée normale de ce cycle lytique. La durée normale du cycle lytique peut être établie expérimentalement (témoin non inhibé) ou ressortir des données de la littérature pour des expériences réalisées dans des conditions expérimentales comparables. De préférence, l'inhibition est de nature à limiter à moins de 20 %, de préférence moins de 10 %, le nombre de bactéries infectées chez lesquelles le phénomène de lyse et de libération des bactériophages est observé.

L'inhibition du cycle lytique est obtenue préférentiellement de manière transitoire, de telle sorte que l'immobilisation du bactériophage dans la bactérie est temporaire, c'est-à-dire limitée dans le temps. De préférence, l'inhibition est réversible. L'invention prévoit en effet la réactivation du cycle après la modification du génome, pour pouvoir récupérer les bactériophages libérés par la bactérie. Les bactériophages modifiés constituent alors la descendance du bactériophage ayant initialement infecté la bactérie et dont le génome a été modifié au cours de l'étape d'immobilisation. Cette réactivation s'opère préférentiellement par une levée de l'inhibition du cycle lytique.

Il n'en reste pas moins que, selon un aspect de l'invention, l'immobilisation du bactériophage peut être prévue sur une très longue durée, notamment en vue de conserver le génome du bactériophage, modifié ou non, sous sa forme immobilisée, dans la bactérie hôte. On peut, par exemple, envisager de congeler les bactéries hôtes après l'étape ii) ou l'étape iii), et ainsi, différer l'étape iv) mentionnée plus haut.

De préférence, le cycle lytique du bactériophage est inhibé à l'étape ii) dans la bactérie hôte, par expression, dans le cytoplasme de la bactérie, d'une protéine ayant pour effet d'interférer avec la phase initiale du cycle de réplication, en particulier de limiter ou empêcher la production des protéines nécessaires à la transcription des gènes du bactériophage. Cette protéine interférente peut être exprimée dans le cytoplasme de la bactérie à l'aide d'un vecteur d'expression, de préférence à partir d'une séquence génétique placée sous le contrôle d'un promoteur inductible. De telles constructions génétiques sont bien connues de l'homme du métier [Sambrook J., Russel D.W. (2001) Molecular Cloning, a Laboratory Manual, CHSL Press]. Des exemples de promoteurs inductibles sont ceux de type GAL inductible par l'adjonction d'IPTG, ou de type HSP, inductible par choc thermique.

De préférence, l'expression de la protéine qui permet l'inhibition du cycle de réplication (cycle lytique) du bactériophage, est induite avant l'infection, c'est-à-dire avant que la bactérie hôte ne soit mise en contact avec le bactériophage.

Selon un aspect préféré de l'invention, le cycle lytique est inhibé à l'aide d'une forme mutée de la protéine Rho, appelée Rho*, ou d'une protéine homologue de celle-ci. La protéine Rho est une protéine présente chez la plupart des bactéries, notamment les bactéries à gram négatif. Par homologue, il faut entendre une protéine ayant les mêmes caractéristiques que Rho, de préférence la même fonction, présentant notamment un pourcentage d'identité en acides aminés supérieur à 40 % à celle-ci, de préférence supérieur à 60 %, plus préférentiellement supérieur à 80 % et encore plus préférentiellement supérieure à 95%.

La protéine Rho et ses différentes formes sont largement décrites dans la littérature [Pinkham,J.L et al. (1983) The nucleotide sequence of the rho gene of E. coli K-12 Nucleic Acids Res. 11(11):3531-3545] [EMBL J01673]. Il est établi que cette protéine, dans sa forme fonctionnelle, est impliquée dans la terminaison de la transcription [Hitchens, T.K. (2006) Sequence specific interactions in the RNA-binding Domain of E. coli Transcription Factor Rho*, J. Biol. Chem. 281 (44) :33697-703]. De nombreuses formes mutantes de cette protéine (Rho*), considérées comme non fonctionnelles, sont décrites dans la littérature. La mutation du gène rho chez *E. coli* a pour effet de diminuer considérablement la croissance des bactéries à tel point qu'il est considéré comme un gène essentiel [Chalissery, J. (2007) Transcription Termination in Defective mutants of Rho : Role of different Functions of Rho in releasing RNA from the Elongation complex, J. Mol. Biol 371 (4) :855-872]. L'absence de protéine Rho fonctionnelle chez *E*. *coli* perturbe le cycle de réplication des bactériophages [Linder, H.C. (1985) E. coli Rho Factor is involved in Lysis of Bacteriophage T4- infected cells, Genetics 111 :197-218]. Cependant de nombreuses questions demeurent sur la manière dont Rho intervient dans le cycle de réplication des bactériophages [Banerjee S. (2006) Rho-dependent Transcription Termination : More questions than answers. J. Microbiol. 44(1) :11-22].

Préférentiellement, c'est une forme mutée Rho*, réputée non fonctionnelle, de la protéine, qui permet d'obtenir l'immobilisation des bactériophages lorsqu'elle est exprimée dans le cytoplasme de la bactérie hôte infectée. Par « non fonctionnelle », il faut entendre une forme mutée de la protéine sauvage, qui ne permet pas d'assurer la fonction qu'elle occupe habituellement dans la cellule. De nombreux mutants de Rho non fonctionnels sont décrits dans la littérature [J. Mol. Biol. (2007) 371(4) :855-872], tels que, par exemple ceux présentant des substitutions dans la séquence sauvage de Rho au niveau des acides aminés suivants: G51V, G53V, Y80C, Y274D, P279S, P279L, G324D, N340S et 1382N.

Dès lors, un aspect préféré de l'invention réside dans la surexpression de protéines Rho* dans une bactérie hôte infectée par un bactériophage afin d'inhiber le cycle lytique dudit bactériophage.

De préférence, cette expression est obtenue à partir d'une copie du gène sauvage présent naturellement dans la bactérie, laquelle copie a été mutée et généralement clonée dans un vecteur d'expression. La copie mutée du gène est de préférence surexprimée en parallèle du gène rho sauvage, lequel est généralement présent sur le chromosome bactérien.

De façon surprenante, les inventeurs ont observé que l'expression d'une protéine Rho* dans la bactérie résultait en l'inhibition du cycle de réplication du bactériophage, quand bien même la protéine Rho sauvage est exprimée dans la bactérie sous une forme fonctionnelle.

A cet égard, l'invention vise plus particulièrement un procédé tel que défini précédemment, comprenant une ou plusieurs les étapes suivantes :
i) on cultive une bactérie hôte possédant un gène rho, ainsi qu'une copie mutée de ce gène (rho*) dont l'expression est facultative;
ii) on induit l'expression du gène rho* muté dans la bactérie hôte ;
iii) on infecte cette bactérie hôte dans laquelle le gène rho* muté est exprimé à l'aide d'un bactériophage lytique qui lui est spécifique, ceci ayant pour effet d'immobiliser le bactériophage dans sa bactérie hôte;
iv) on modifie le génome du bactériophage à l'intérieur de la bactérie hôte durant l'étape iii);
v) on cesse d'induire l'expression de rho* en laissant le gène rho sauvage s'exprimer dans la bactérie, ce qui a pour effet de lever l'immobilisation du bactériophage et libérer sa progéniture, dont le génome a été modifié.

Il est avantageux selon l'invention de conserver une copie du gène rho sauvage fonctionnelle dans la bactérie hôte, parallèlement à la copie mutée rho*. Il apparait, en effet, que l'expression de la protéine Rho [+] permet à la bactérie de continuer à se diviser durant la durée d'immobilisation du bactériophage. On peut ainsi laisser la bactérie hôte se diviser tandis que le bactériophage est immobilisé, notamment entre les étapes iii) et iv) du procédé. Les inventeurs ont ainsi vu que tant qu'il était immobilisé, le génome du bactériophage était répliqué en même temps que celui de son hôte. Il est de ce fait possible d'augmenter le nombre de cellules dans lesquelles les bactériophages sont immobilisés par division des bactéries hôtes, et ainsi augmenter facilement le nombre de bactéries dans lesquelles les bactériophages sont immobilisés au cours du procédé.

Sans être liés par la théorie, les inventeurs ont émis l'hypothèse que la protéine Rho* avait la capacité d'entrer en compétition avec la protéine Rho sauvage.

Cependant, selon les connaissances actuelles, il est difficile d'évaluer si la protéine Rho intervient spécifiquement dans le cycle lytique du bactériophage ou plus généralement si elle agit sur l'ensemble des mécanismes de duplication (ou de transcription) des éléments génétiques présents dans la bactérie.

Un avantage supplémentaire de l'invention réside dans le fait qu'au cours de la phase d'immobilisation du bactériophage, la bactérie hôte reste apte à recevoir des séquences d'acides nucléiques d'origine exogène. En particulier, il est possible de faire pénétrer dans leur cytoplasme, par exemple, des fragments de PCR, des ADNc, des ARN ou des vecteurs, et ainsi transformer génétiquement ces bactéries, notamment par électroporation, c'est à dire en appliquant un champ électrique. Cette méthode est bien connue de l'homme du métier [Dunny, G. M. et al. (1991) Improved electroporation and cloning system for gram-positive bacteria. Appl. Environ. Microbiol. 57:1194-1201] [Reysset, G. (1993) Transformation and electrotransformation in clostridia, p. 111-119. In M. Sebald (ed.), Genetics and molecular biology of anaerobes. Springer-Verlag, New York N.Y] [Sambrook, J. and D. W. Russell (2001). Molecular cloning: a laboratory manual, 3rd ed. Cold Spring Harbor Laboratory Press, Plainview, N.Y.]. Un aspect de l'invention réside donc dans le fait de pouvoir modifier le génome du bactériophage au cours de son immobilisation, par transformation de la bactérie hôte. De préférence, la transformation se produit au moyen d'un ou plusieurs vecteurs, notamment un vecteur pour la recombinaison homologue.

La transformation de la bactérie hôte peut intervenir avant ou pendant l'étape iii) correspondant à la phase d'immobilisation. En particulier, la bactérie hôte peut être transformée à l'aide d'un ADN ou ARN de nature exogène avant d'être infectée par le bactériophage.

Selon un aspect de l'invention, tout ou partie du génome du bactériophage peut être inséré dans un vecteur par recombinaison homologue présent dans la bactérie, au cours de la phase d'immobilisation, comme indiqué dans les exemples.

Un autre aspect de l'invention concerne la préparation d'une bactérie hôte permettant la mise en oeuvre du procédé décrit précédemment. Une telle bactérie hôte consiste plus particulièrement en une bactérie comprenant une copie mutée du gène rho (rho *), permettant l'expression de protéines Rho*, présente de préférence sur un vecteur d'expression. En général, la bactérie hôte selon l'invention comprend, en outre, une copie du gène rho sur son chromosome permettant l'expression de protéines Rho sauvages.

Lorsque ladite bactérie hôte est infectée par un bactériophage, celle-ci comprend, en outre, dans son cytoplasme, le génome dudit bactériophage.

Une bactérie hôte préférée selon l'invention est plus particulièrement une bactérie (Rho* - λ) telle que décrite dans les exemples de la présente demande.

La présente demande a également pour objet un kit pour la mise en oeuvre du procédé selon l'invention, comprenant un ou plusieurs éléments selon l'invention choisis parmi les suivants :
- une bactérie hôte telle que définie précédemment,
- un vecteur d'expression comprenant une copie du gène rho muté (rho*), de préférence placée sous contrôle d'un promoteur inductible,
- un vecteur de recombinaison homologue permettant de modifier le génome du bactériophage,
- des réactifs ou milieux de culture permettant de cultiver la bactérie hôte et d'induire l'immobilisation du bactériophage dans ladite bactérie hôte.

La présente invention est particulièrement adaptée à la production de bactériophages dont les protéines de ciblages sont modifiées.

En particulier, l'invention prévoit que le génome d'un bactériophage soit modifié à l'étape iii) du procédé décrit précédemment au niveau d'un gène exprimant une protéine de ciblage, de préférence un gène codant les protéines GP12, GP36, GP37 et GP38 ou des protéines homologues de celles-ci.

Les protéines de ciblage du bactériophage se définissent comme des protéines qui participent à la reconnaissance et à l'adhésion du bactériophage à la bactérie hôte. Ces protéines sont de préférence choisies parmi celles constituant les fibres de queue, la plate-forme basale ou la capside du bactériophage T4. Une protéine particulièrement visée par le procédé selon l'invention est la protéine GP12 de la plate forme basale dont la séquence nucléotidique est disponible dans la base de données Swissprot (Uniprot) sous le N° d'accession [P10930]. D'autres protéines de ciblage préférées sont GP36 [P03743], GP37 [P03744] et GP38 [P03739], que l'on retrouve dans les régions distales des fibres de queue, ou bien encore les protéines de la capside GP23 [P04535], GP24 [P19896], Hoc [P18056] et Soc [P03715].

Bien entendu, des protéines homologues de celles citées ci-dessus, présentes chez d'autres bactériophages que les bactériophages de type-T sont également préférées.

Par séquence homologue, il faut entendre des protéines ayant une séquence d'acides aminés présentant au moins 50 % d'identité avec ces dernières, de préférence au moins 70 %, plus préférentiellement au moins 90%.

Selon l'invention, les bactériophages sont modifiés au niveau des gènes codant les protéines de ciblage par insertion de séquences oligonucléotidiques produites de manière aléatoire.

Le procédé selon l'invention se montre particulièrement utile pour pouvoir insérer, par recombinaison homologue, des fragments de PCR, lesquels peuvent être introduits sous forme monobrin par électroporation.

La période d'immobilisation du bactériophage dans la bactérie hôte est en effet propice à l'intégration de tels fragments, à condition toutefois que lesdits fragments comprennent, au moins en partie, des séquences homologues à celles du génome du bactériophage.

A cet égard, il sera fait référence au procédé de PCR décrit dans une précédente demande du demandeur WO 2008/093010, lequel permet de générer un grand nombre de copies de fragments d'ADN comprenant, pour partie, des séquences aléatoires, et pour autre partie, des séquences homologues conservées permettant de cibler les gènes du bactériophage. Ces fragments d'ADN peuvent avoir une taille de 20 à 4000 kb, de préférence de 30 à 2000 kb, et plus préférentiellement de 40 à 100 kb.

Le procédé selon l'invention permet d'utiliser directement une grande diversité de produits PCR et donc de s'affranchir de l'étape souvent incontournable du clonage de ces fragments. Toutefois, de tels fragments peuvent être également clonés sur des vecteurs de recombinaison homologue sans affecter le procédé selon l'invention.

Selon un aspect préféré de l'invention, plusieurs gènes codant des protéines de ciblage du bactériophage sont modifiés simultanément par recombinaison homologue au cours de la phase d'immobilisation du bactériophage selon l'étape iii) du procédé décrit plus haut. Pour parvenir à un tel résultat, l'invention prévoit notamment de transformer la bactérie hôte successivement ou de manière concomitante, par électroporation de fragments d'ADN différents, ciblant, de préférence, des gènes différents.

Des vecteurs préférés permettant de modifier les gènes du bactériophage T4 selon l'invention dans la bactérie hôte *E. coli* sont, par exemple, les vecteurs pACYC184 (ATCC 37033), pBAD18-K (ATCC 87397) et RR1 (ATCC 87076). De tels vecteurs présentent l'avantage de posséder des marqueurs conférant une résistance à des antibiotiques différents. En outre, ils ne partagent pas de séquences nucléotidiques communes susceptibles d'occasionner des recombinaisons entre les différents vecteurs, une fois ceux-ci intégrés dans la bactérie hôte.

Selon l'invention, la modification des bactériophages s'effectue au cours d'un seul cycle lytique, dont la durée est artificiellement prolongée par immobilisation du bactériophage dans la bactérie hôte.

La descendance des bactériophages, obtenue à l'issue de ce cycle lytique, comprend généralement plus de 10%, de préférence plus de 20 %, plus préférentiellement plus de 50 % de bactériophages génétiquement modifiés. Cette descendance peut être réutilisée pour un second cycle de réinfection.

Avantageusement, dans le cas de la modification des protéines de ciblage des bactériophages, les bactériophages modifiés sont recueillis à l'issue du premier cycle lytique. En effet, en se limitant à un seul cycle de réplication, les bactériophages présentent une diversité maximale, laquelle serait moindre si des cycles de réinfection ultérieurs étaient pratiqués.

Une banque de bactériophages selon l'invention comprend des bactériophages dont une ou plusieurs protéines de ciblage sont diversifiées. De tels bactériophages peuvent reconnaître potentiellement de nouvelles souches bactériennes. Ils représentent un moyen potentiel de lutte à l'encontre de bactéries émergentes ou de souches devenues résistantes aux antibiotiques.

Un aspect de l'invention consiste en l'utilisation de ces bactériophages génétiquement modifiés en tant qu'agents antibactériens, notamment dans le domaine médical, pour le traitement d'infections bactériennes.

D'une manière plus générale, l'invention vise un génome de bactériophage modifié selon le procédé de la présente invention, ainsi que les bactériophages génétiquement modifiés susceptibles d'en résulter.

L'invention a également pour objet les protéines modifiées, pouvant être extraites des bactériophages modifiés selon l'invention, ou exprimées de manière recombinante à partir de l'ADN provenant de ce bactériophage ou de la bactérie hôte dans laquelle il a été immobilisé. Une telle protéine modifiée est préférentiellement une protéine de ciblage du bactériophage.

Les exemples qui suivent ont pour but d'illustrer l'invention sans en limiter la portée.

### EXEMPLES

### 1/ Construction d'une bactérie hôte (N°1) DK8-rho*-λ pour « immobiliser » le bactériophage T4

### Etape 1 : Préparation du gène rho muté dans un vecteur d'expression.

Une copie du gène rho est amplifiée par PCR à partir de gènes rho* mutés d'*E.coli* codant respectivement les protéines Rho* G51 V, Y80C et Y274D [J. Mol. Biol. (2007) 371 (4) :855-872]. L'amplification est réalisée à l'aide des amorces suivantes :
Rho F: 5' CACCATGAATCTTACCGAATTAAAGAATACG 3' (SEQ ID NO.1)
Rho R :5' TTATGAGCGTTTCATCATTTCGA 3' (SEQ ID NO.2)

Après purification sur un gel d'agarose préparatif, le produit de PCR (Rho*) est cloné dans le vecteur d'expression pHSG299 sous contrôle d'un promoteur *lac* inductible par l'IPTG, en utilisant les enzymes de restriction *Eco*RI et *Sal*l. Après ligation, le vecteur pHSG299rho* est purifié, remis en suspension dans ddH2O et sert à transformer des cellules DK8 (ATCC 47038) « électrocompétentes ». Les transformants sont sélectionnés en milieu LB contenant 30 µg/ml de kanamycine et sont cultivés pendant une nuit à 30 °C. Quelques colonies sont prélevées pour la vérification par PCR de la présence du segment d'insertion *rho**. Les colonies positives sont cultivées dans du milieu LB + Km pour la préparation d'une culture concentrée de cellules DK8-Rho*.

### Etape 2 : Construction de bactérie hôte E. coli « mini-λ ».

Pour obtenir la recombinaison efficace de l'ADN donneur dans des fonds *recA⁺* ou *recA⁻,* des bactéries hôte DK8 d'*E*. *coli* contenant un prophage λ porteur des gènes de recombinaison *exo, bet* et *gam* sous le contrôle d'un répresseur *cl* de λ sensible à la température sont préparées. Les gènes *exo*, *bet* et *gam* sont activés à 42 °C et réprimés à 32 °C. Lorsque les fonctions de λ sont actionnées pendant un temps réduit à 5 min, les cellules deviennent davantage recombinogènes et absorbent l'ADN linéaire sans destruction. La protéine Gam codée par λ inhibe l'attaque de l'ADN linéaire par la nucléase *Rec*BCD d'*E.coli*, tandis qu'Exo et Beta engendrent une activité de recombinaison pour cet ADN linéaire. Cette recombinaison est beaucoup plus efficace pour des homologies d'ADN limitées à 30 à 50 pb aux extrémités des ADN linéaires.

Les oligonucléotides 5' GTATGCATGCTGGGTGTGG (MλRf) (SEQ ID NO.3) et 5' CGCACTCTCGATTCGTAGAGCCTCG (MλRr) (SEQ ID NO.4) sont utilisés comme amorces pour l'amplification du prophage λ cl857.

Une fois le prophage λ amplifié par PCR, il est cloné, au niveau du site *Sma*l (extrémité franche), dans le plasmide pFN476 (ATCC86962), qui est un plasmide à nombre réduit de copies, contenant le promoteur *lac* inductible par l'IPTG. Après ligation, l'ADN est purifié, remis en suspension dans ddH2O et transformé dans les cellules DK8-*rho** précédemment obtenues par électroporation [Sambrook, J. and D. W. Russell (2001) Molecular cloning: a laboratory manual, 3rd ed. Cold Spring Harbor Laboratory Press, Plainview, N.Y.) Après rétablissement, les cellules sont étalées sur des plaques de milieu LB X-gal + Kan + Amp et mises en incubation à 30 °C.

Quelques colonies blanches sont sélectionnées pour la vérification par PCR de la présence du prophage λ. Une colonie positive est ensuite cultivée pendant une nuit à 30 °C dans du milieu LB X-Gal + Kan + Amp pour préparer une culture concentrée de cellules (DK8-*rho**-λ).

A cette étape, les hôtes transformés sont inductibles par λ à 42°C (température élevée), *lac*Z-positifs et contiennent des copies du gène *rho*.*

### 2/ Immobilisation du bactériophage T4 dans la bactérie hôte N°1

Une culture fraîche de cellules DK8- *rho*-λ* est cultivée pendant une nuit dans du milieu LB X-Gal + Kan + Amp à 30 °C.

Les cultures pour infection par T4 sont commencées avec un volume égal ou inférieur à 0,05 ml de cellules issues d'une culture d'une nuit pour 10 ml de milieu LB + Kan + Amp afin de garantir que les cellules passent à la phase de croissance exponentielle avant l'addition du bactériophage.

Pour améliorer l'aération, ces cultures sont multipliées dans des fioles d'Erlenmeyer de 250 ml à branche latérale, bouchées non hermétiquement, dans un bain d'eau d'agitateur par secousses à 30 °C.

250 ml de cellules dans du milieu LB X-Gal + Kan + Amp avec 1mM d'IPTG (inducteur d'expression de rho*) sont cultivés à une densité de 3 x 10⁸ cellules par ml à 30 °C avec agitation par secousses.

Le bactériophage T4 est ajouté à une multiplicité d'environ 10 particules par cellule et la croissance se poursuit pendant exactement 20 min.

Les cellules sont étalées des plaques de milieu LB X-gal + Kan + Amp + IPTG et mises en incubation à 30 °C.

Quelques colonies sont sélectionnées pour la vérification par PCR de la présence de l'ADN du bactériophage T4 (cf. Figure 2). Une colonie positive de bactériophage T4 est ensuite cultivée pendant une nuit à 30 °C dans du milieu LB X-Gal + Kan + Amp + IPTG pour préparer une culture concentrée de cellules (DK8-Rho*-λ-DNAT4) à utiliser dans les manipulations ultérieures.

### 3/ Production n°1 de la descendance modifiée du bactériophage T4

Les cellules DK8-Rho*-λ-DNA T4 sont cultivées en milieu liquide, puis transformées par électroporation selon le protocole usuel [ Sambrook, J. and D. W. Russell (2001) Molecular cloning: a laboratory manual, 3rd ed. Cold Spring Harbor Laboratory Press, Plainview, N.Y.] à l'aide de fragments d'ADN issus directement d'une PCR purifiée. Les fragments de PCR consistent en des fragments modifiés des gènes *gp37, gp12* et *gp38* décrits dans WO 2008/093010.

Après transformation, les bactéries transformées sont cultivées sur du milieu LB X-Gal + Kan + Amp+ IPTGpréchauffé à 42 °C. Les cellules sont mises en incubation pendant exactement 15 min à 42 °C avec aération constante. Les cultures sont transférées ensuite à un bain d'eau à 30 °C, en laissant la croissance se poursuivre pendant exactement 25 min.

Le but de cette étape consiste à induire une recombinaison homologue entre les fragments PCRs introduits et l'ADN du bactériophage T4 présent dans la bactérie.

Une fois la recombinaison effectuée, les cellules DK8- Rho*-λ-DNA sont remises en suspension et lavées en milieu LB + Kan + Amp, puis mises en incubation pendant exactement 2 heures à 30 °C. Durant cette incubation, les bactéries sont lysées et libèrent la descendance du bactériophage T4. Cette descendance est composée, en partie, de bactériophages modifiés.

Les bactéries lysées sont recueillies par centrifugation à 5000 tr/min pendant 5 minutes tandis que le surnageant de lyse est récupéré. Quelques gouttes de chloroforme sont ajoutées au surnageant, puis celui-ci est centrifugé, de nouveau, pendant 10 min à 6000 tr/min. Le chloroforme est éliminé. Puis, le surnageant, qui contient les bactériophages modifiés, est ajusté à une concentration en tampon SM 1 X (MgSO4 10 mM, NaCl 100 mM, 0,01 % de gélatine et Tris-HCl 50 mM [pH 7,5]) en utilisant du tampon concentré 5X, pour être stocké à 4 °C.

### 4/ Construction d'une bactérie hôte (N°2) pour « immobiliser » le bactériophage T4 sur un plasmide

Le but de cette construction est de cloner le génome du bactériophage T4 dans un vecteur, une fois ledit génome immobilisé dans la bactérie.

L'insertion dans le vecteur s'effectue par recombinaison homologue au niveau de la séquence du gène *mobA* présente dans le génome du bactériophage.

### Etape 1 : Préparation d'un vecteur comprenant une copie du gène mobA du bactériophage T4.

Le gène *mobA* est amplifié par PCR à partir de l'ADN génomique du bactériophage T4 en utilisant les amorces suivantes:
*mob*A F :5' GTAGAAAATAGTGCTAAAAAGTGT 3' (SEQ ID NO.5)
*mob*A R :5' TTAATAGTGCGGGGTAAAACCC 3' (SEQ ID NO.6)

Après purification sur un gel d'agarose préparatif, le produit de PCR *(mobA)* est cloné entre deux sites loxP du vecteur pDNR-1r au niveau du site de coupure *Sma*l. Des cellules DK8 (ATCC 47038) d'*E.coli* sont ensuite transformées à l'aide du vecteur pDNR-1*rmob*A qui comporte un gène de résistance au chloramphénicol (Cm). Les transformants sont sélectionnés en milieu LB contenant 30 µg/ml de chloramphénicol à 37 °C. Après vérification, les transformants (DK8 - *mob*A) sont utilisés pour préparer un stock de vecteur pDNR-1r*mob*A.

### Etape 2 : Introduction du vecteur pDNR-1rmobA dans cellules DK8-Rho* - λ.

Les cellules DK8-Rho*-λ sont rendues « électrocompétentes » selon le protocole rappelé au point 3/ ci-dessus, puis transformées à l'aide du vecteur pDNR-1 r*mob*A préparé ci-dessus.

Les cellules transformées sont étalées sur des plaques de milieu LB X-Gal + Kan + Amp + Cm, puis mises en incubation à 30 °C.

Quelques colonies sont sélectionnées pour vérification par PCR de la présence du vecteur pDNR-1*rmob*A. Une colonie positive est ensuite cultivée pendant une nuit à 30 °C dans du milieu LB X-Gal + Kan + Amp + Cm pour préparer une culture concentrée de cellules (DK8-Rho*-λ-mobA) utilisable dans les manipulations ultérieures.

### 5/ Immobilisation du bactériophage T4 dans la bactérie hôte N°2

Une culture fraîche d'une nuit de cellules DK8-Rho*-λ-mobA est préparée dans du milieu LB X-Gal + Kan + Amp + Cm à 30 °C.

Les cultures, destinées à être infectées par le bactériophage T4, sont commencées avec un volume égal ou inférieur à 0,05 ml de cellules d'une culture d'une nuit pour 10 ml de milieu LB X-Gal + Kan + Amp + Cm afin de garantir que les cellules passent à la phase de croissance exponentielle avant l'addition dudit bactériophage.

Pour améliorer l'aération, ces cultures sont multipliées dans des fioles d'Erlenmeyer de 250 ml à branche latérale, bouchées non hermétiquement, dans un bain d'eau d'agitateur par secousses à 30 °C.

250 ml de cellules dans du milieu LB X-Gal + Kan + Amp + Cm sont cultivées avec l'inducteur d'expression de Rho* IPTG sont cultivés à une densité de 3 x 108 cellules par ml à 30 °C avec agitation par secousses.

Des portions aliquotes de 10 ml de cellules à croissance exponentielle sont ensuite transférées à 40 ml de milieu LB X-Gal + Kan + Amp + Cm préchauffé à 42 °C et mises en incubation pendant exactement 15 min à 42 °C avec aération constante. Le bactériophage T4 est ajouté à une multiplicité d'environ 10 particules par cellule. Les cultures sont transférées à un bain d'eau à 30 °C, en laissant la croissance se poursuivre pendant exactement 25 min.

Les cellules sont étalées des plaques de milieu LB X-Gal+ Kan + Amp + Cm + IPTG et mises en incubation à 30 °C.

Quelques colonies sont sélectionnées pour la vérification par PCR de la présence de l'ADN du bactériophage T4. Une colonie positive de bactériophage T4 est ensuite cultivée pendant une nuit à 30 °C dans du milieu LB X-Gal+ Kan + Amp + Cm + IPTG pour préparer une culture concentrée de cellules (DK8-Rho*-λ-mobA -DNA T4) à utiliser dans les manipulations ultérieures.

### 6/ Production n°2 de la descendance du bactériophage T4 avec des modifications

Afin de libérer le génome du bactériophage du plasmide pDNR-1 r*mob*A, un autre plasmide pHSG-cre (ATCC n°87075) exprimant la protéine Cre est introduit dans des cellules DK8-Rho*-λ-mobA-DNA T4. Ce plasmide est introduit en suivant le même protocole d'électroporation que précédemment. L'induction de Rho* dans la bactérie hôte est arrêtée. Pour ce faire, les cellules sont centrifugées, le milieu est retiré et le culot bactérien est lavé avec du milieu LB sans inducteur. L'expression de la protéine Cre entraine alors une recombinaison au niveau des sites loxP du vecteur pDNR-1 r*mob*A -T4, et ainsi, la libération de l'ADN génomique complet du bactériophage T4.

Les cellules sont alors mises en incubation pendant exactement 2 heures à 30 °C en LB X-Gal + Kan + Amp+ Cm. Durant cette incubation, les bactériophages reprennent leur cycle lytique : les bactéries sont lysées et libèrent la progéniture du bactériophage T4.

Les bactériophages sont récupérés par centrifugation de la même manière qu'au point 3/.

## Revendications

1. Procédé pour modifier le génome d'un bactériophage lytique dans une bactérie hôte, **caractérisé en ce que** :
(i) on infecte une bactérie hôte avec un bactériophage ;
(ii) on inhibe le cycle lytique du bactériophage dans la bactérie hôte;
(iii) on modifie le génome du bactériophage pendant l'étape ii) durant laquelle le bactériophage est immobilisé dans la bactérie hôte ;
(iv) on lève l'inhibition de l'étape ii) du cycle lytique, de sorte à libérer la progéniture du bactériophage de l'étape (i) dont le génome a été modifié.

2. Procédé selon la revendication 1, **caractérisé en ce que** le bactériophage lytique est un bactériophage de la famille des Myoviridae, tel qu'un bactériophage de la famille T, de préférence un bactériophage de type T4.

3. Procédé selon les revendications 1 ou 2, **caractérisé en ce que** le cycle lytique du bactériophage est inhibé dans la bactérie hôte à l'étape ii) par surexpression, dans le cytoplasme de la bactérie, de la protéine Rho de la bactérie, sous une forme non-fonctionnelle (Rho*).

4. Procédé selon la revendication 3, en ce que les protéines Rho* sont surexprimées à l'aide d'une copie mutée du gène rho sous contrôle d'un promoteur inductible.

5. Procédé selon la revendication 4, **caractérisé en ce que** la bactérie hôte à l'étape iii) subit une étape de transformation de la bactérie, notamment par l'action d'un champ électrique (éléctroporation), afin que celle-ci intègre des séquences d'acides nucléiques exogènes.

6. Procédé selon la revendication 5, **caractérisé en ce que** lesdites séquences d'acides nucléiques consistent en des séquences oligonucléotidiques produites par PCR ou des vecteurs de recombinaison homologue.

7. Procédé selon la revendication 5 ou 6, **caractérisé en ce que** la modification du génome du bactériophage à l'étape iii) dans la bactérie hôte s'effectue par recombinaison homologue à l'aide d'un vecteur, après transformation de la cellule hôte par ce vecteur.

8. Procédé selon l'une des revendications 1 à 7, **caractérisé en ce que** le génome du bactériophage est intégré dans un vecteur de réplication au cours de l'étape iii).

9. Procédé selon l'une des revendications 1 à 8, **caractérisé en ce qu'**on laisse la bactérie hôte se diviser tandis que le bactériophage y est immobilisé entre les étapes ii) et iv).

10. Procédé selon l'une des revendications 1 à 9, **caractérisé en ce qu'**il comprend les étapes suivantes :
i) on cultive une bactérie hôte possédant un gène rho, ainsi qu'une copie mutée de ce gène (*rho**) dont l'expression est facultative ;
ii) on induit l'expression du gène rho* muté dans la bactérie hôte ;
iii) on infecte cette bactérie hôte dans laquelle le gène *rho** muté est exprimé à l'aide d'un bactériophage lytique qui lui est spécifique, ceci ayant pour effet d'immobiliser le bactériophage dans sa bactérie hôte ;
iv) on modifie le génome du bactériophage à l'intérieur de la bactérie hôte durant l'étape iii) ;
v) on cesse d'induire l'expression de *rho** en laissant le gène *rho* sauvage s'exprimer dans la bactérie, ce qui a pour effet de lever l'immobilisation du bactériophage et libérer la progéniture du bactériophage dont le génome a été modifié.

11. Procédé selon l'une des revendications 1 à 10, **caractérisé en ce que** le génome dudit bactériophage est modifié à l'étape iii) au niveau d'un gène exprimant une protéine de ciblage.

12. Procédé selon la revendication 11, **caractérisé en ce que** ladite protéine de ciblage est choisie parmi GP12, GP36, GP37 et GP38 ou une protéine homologue de celles-ci.

13. Procédé selon l'une des revendications 1 à 10, **caractérisé en ce que** le génome dudit bactériophage est modifié à l'étape iii) au niveau d'un gène exprimant une protéine de la capside du bactériophage.

14. Procédé selon la revendication 13, **caractérisé en ce que** ladite protéine de capside est choisie parmi GP23, GP24, Hoc et Soc ou une protéine homologue de celles-ci.

15. Bactérie hôte pour la mise en oeuvre du procédé selon l'une quelconque des revendications 1 à 14, **caractérisée en ce qu'**elle comprend :
- une copie du gène *rho* de ladite bactérie permettant l'expression de protéines Rho fonctionnelles;
- un vecteur comprenant une copie mutée dudit gène rho (*rho* *), permettant l'expression de protéines Rho* ;
- le génome d'un bactériophage lytique.

16. Bactérie selon la revendication 15, **caractérisée en ce qu'**elle comprend, en outre, un vecteur de recombinaison homologue comprenant une ou plusieurs régions homologues au génome dudit bactériophage lytique.

## Claims

1. Method for modifying the genome of a lytic bacteriophage in a host bacterium, **characterized in that**:
(i) a host bacterium is infected with a bacteriophage;
(ii) the lytic cycle of the bacteriophage is inhibited in the host bacterium;
(iii) the bacteriophage genome is modified during step ii) during which the bacteriophage is immobilized in the host bacterium;
(iv) the inhibition of step ii) of the lytic cycle is removed, so as to release the progeny of the bacteriophage of step (i), the genome of which was modified.

2. Method according to claim 1, **characterized in that** the lytic bacteriophage is a bacteriophage of the Myoviridae family, such as a bacteriophage of the T family, preferably a bacteriophage of the T4 type.

3. Method according to claims 1 or 2, **characterized in that** the lytic cycle of the bacteriophage is inhibited in the host bacterium in step ii) by overexpression, in the cytoplasm of the bacterium, of the Rho protein of the bacterium, in a non-functional form (Rho*).

4. Method according to claim 3, **characterized in that** Rho* proteins are overexpressed by means of a mutated copy of the *rho* gene under the control of an inducible promoter.

5. Method according to claim 4, **characterized in that** the host bacterium in step iii) undergoes a step of bacterium transformation, in particular by the action of an electric field (electroporation), so that the latter integrates exogenous nucleic acid sequences.

6. Method according to claim 5, **characterized in that** said nucleic acid sequences consist of oligonucleotide sequences produced by PCR or homologous recombination vectors.

7. Method according to claim 5 or 6, **characterized in that** modification of the bacteriophage genome in step iii) in the host bacterium is carried out by homologous recombination by means of a vector, after transformation of the host cell by this vector.

8. Method according to one of claims 1 to 7, **characterized in that** the bacteriophage genome is integrated in a replication vector during step iii).

9. Method according to one of claims 1 to 8, **characterized in that** the host bacterium is allowed to divide whereas the bacteriophage is immobilized therein between steps ii) and iv).

10. Method according to one of claims 1 to 9, **characterized in that** it comprises the following steps:
i) culturing a host bacterium possessing a *rho* gene, as well as a mutated copy of said gene (rho*), the expression of which is optional;
ii) inducing the expression of the mutated rho* gene in the host bacterium;
iii) infecting said host bacterium in which the mutated rho* gene is expressed by means of a lytic bacteriophage specific to it, this having the effect of immobilizing the bacteriophage in its host bacterium;
iv) modifying the bacteriophage genome within the host bacterium during step iii);
v) ceasing induction of the expression of *rho*,* allowing the wild-type *rho* gene to be expressed in the bacterium, which has the effect of removing the immobilization of the bacteriophage and releasing the progeny of the bacteriophage, the genome of which was modified.

11. Method according to one of claims 1 to 10, **characterized in that** the genome of said bacteriophage is modified in step iii) at a gene expressing a targeting protein.

12. Method according to claim 11, **characterized in that** said targeting protein is selected from GP12, GP36, GP37 and GP38 or a protein that is homologous with these latter.

13. Method according to one of claims 1 to 10, **characterized in that** the genome of said bacteriophage is modified in step iii) at a gene expressing a capsid protein of the bacteriophage.

14. Method according to claim 13, **characterized in that** said capsid protein is selected from GP23, GP24, Hoc and Soc or a protein that is homologous with these latter.

15. Host bacterium for implementing the method according to any one of claims 1 to 14, **characterized in that** it comprises:
- a copy of the *rho* gene of said bacterium permitting the expression of functional Rho proteins;
- a vector comprising a mutated copy of said rho gene (*rho**), permitting the expression of Rho* proteins;
- the genome of a lytic bacteriophage.

16. Bacterium according to claim 15, **characterized in that** it further comprises a homologous recombination vector comprising one or more regions homologous with the genome of said lytic bacteriophage.

## Patentansprüche

1. Verfahren zur Modifikation des Genoms eines lytischen Bakteriophagen in einem Wirtsbakterium, **dadurch gekennzeichnet, dass**:
(i) ein Wirtsbakterium mit einem Bakteriophagen infiziert wird;
(ii) der lytische Zyklus des Bakteriophagen in dem Wirtsbakterium gehemmt wird;
(iii) das Genom des Bakteriophagen während des Schrittes ii), in welchem der Bakteriophage in dem Wirtsbakterium immobilisiert wird, modifiziert wird;
(iv) die Hemmung des lytischen Zyklus des Schrittes ii) aufgehoben wird, um so die Nachkommen des Bakteriophagen des Schrittes (i), dessen Genom modifiziert wurde, freizugeben.

2. Verfahren nach Anspruch 1, **dadurch gekennzeichnet, dass** der lytische Bakteriophage ein Bakteriophage der Familie Myoviridae, wie etwa ein Bakteriophage der T-Familie, vorzugsweise ein Bakteriophage des Typs T4, ist.

3. Verfahren nach den Ansprüchen 1 oder 2, **dadurch gekennzeichnet, dass** der lytische Zyklus des Bakteriophagen in dem Wirtsbakterium in Schritt ii) durch Überexpression des Rho-Proteins des Bakteriums in einer nicht-funktionellen Form (Rho*) in das Zytoplasma der Bakterien gehemmt wird.

4. Verfahren nach Anspruch 3, wobei die Rho*-Proteine unter Verwendung einer mutierten Kopie des Gens *rho* unter der Kontrolle eines induzierbaren Promotors überexprimiert werden.

5. Verfahren nach Anspruch 4, **dadurch gekennzeichnet, dass** das Wirtsbakterium in dem Schritt iii) einem Transformationsschritt des Bakteriums, insbesondere durch die Wirkung eines elektrischen Feldes (Elektroporation), unterzogen wird, so dass es exogene Nukleinsäuresequenzen umfasst.

6. Verfahren nach Anspruch 5, **dadurch gekennzeichnet, dass** die Nukleinsäuresequenzen aus durch PCR oder aus homologen Rekombinationsvektoren erzeugten Oligonukleotid-Sequenzen bestehen.

7. Verfahren nach Anspruch 5 oder 6, **dadurch gekennzeichnet, dass** die Modifikation des Bakteriophagengenoms in Schritt dem iii) in dem Wirtsbakterium durch homologe Rekombination unter Verwendung eines Vektors nach der Transformation der Wirtszelle mit diesem Vektor bewirkt wird.

8. Verfahren nach einem der Ansprüche 1 bis 7, **dadurch gekennzeichnet, dass** das Genom des Bakteriophagens während des Schrittes iii) in einen Replikationsvektor integriert wird.

9. Verfahren nach einem der Ansprüche 1 bis 8, **dadurch gekennzeichnet, dass** das Wirtsbakterium sich teilen gelassen wird, während der Bakteriophage zwischen den Schritten ii) und iv) immobilisiert wird.

10. Verfahren nach einem der Ansprüche 1 bis 9, **dadurch gekennzeichnet, dass** es die folgenden Schritte umfasst, dass:
i) ein Wirtsbakterium kultiviert wird, das ein Gen rho sowie eine mutierte Kopie dieses Gens (*rho**) aufweist, dessen Expression fakultativ ist;
ii) die Expression des mutierten Gens rho* in dem Wirtsbakterium induziert wird;
iii) dieses Wirtsbakterium, in welchem das mutierte Gen *rho** exprimiert ist, unter Verwendung eines lytischen Bakteriophagen, der für dieses spezifisch ist, infiziert wird, was die Wirkung einer Immobilisierung des Bakteriophagen in seinem Wirtsbakterium hat;
iv) das Genom des Bakteriophagen im Inneren des Wirtsbakteriums während des Schrittes iii) modifiziert wird;
v) aufgehört wird, die Expression von *rho** zu induzieren, indem das Wildtyp-Gen *rho* sich in dem Bakterium exprimieren gelassen wird, was die Wirkung des Aufhebens der Immobilisierung des Bakteriophagen und der Freisetzung der Nachkommen des Bakteriophagen, dessen Genom modifiziert wurde, hat.

11. Verfahren nach einem der Ansprüche 1 bis 10, **dadurch gekennzeichnet, dass** das Genom des Bakteriophagen in dem Schritt iii) auf das Niveau eines Gens, das ein Zielprotein exprimiert, modifiziert wird.

12. Verfahren nach Anspruch 11, **dadurch gekennzeichnet, dass** das Zielprotein aus GP12, GP36, GP37 und GP38 oder einem homologen Protein derselben ausgewählt wird.

13. Verfahren nach einem der Ansprüche 1 bis 10, **dadurch gekennzeichnet, dass** das Genom des Bakteriophagen in dem Schritt iii) auf
das Niveau eines Gens, das ein Capsid-Protein des Bakteriophagen exprimiert, modifiziert wird.

14. Verfahren nach Anspruch 13, **dadurch gekennzeichnet, dass** das Capsid-Protein aus GP23, GP24, Hoc und Soc oder einem homologen Protein derselben ausgewählt wird.

15. Wirtsbakterium für die Durchführung des Verfahrens nach einem der Ansprüche 1 bis 14, **dadurch gekennzeichnet, dass** es umfasst:
- eine Kopie des Gens *rho* des Bakteriums, das die Expression von funktionellen Rho-Proteinen gestattet;
- einen Vektor, der eine mutierte Kopie des Gens rho (*rho**) umfasst, das die Expression von Rho*-Proteinen gestattet;
- das Genom eines lytischen Bakteriophagen.

16. Bakterium nach Anspruch 15, **dadurch gekennzeichnet, dass** es ferner einen homologen Rekombinationsvektor mit einem oder mehreren homologen Bereichen zu dem Genom des lytischen Bakteriophagen umfasst.


### Listage de séquences

<110> PHERECYDES PHARMA
   <120> Modification du génome d\222un bactériophage lytique par immobilisation dudit bactériophage dans sa bactérie hôte
<130> B090081QTA
<140> FR 0952933
   <141> 2009-04-30
<160> 6
<210> 1
   <211> 31
   <212> DNA
   <213> Artificial sequence
<220> primer_bind
   <223> RhoF primer for amplification of rho gene
<400> 1
   caccatgaat cttaccgaat taaagaatac g
   31
<210> 2
   <211> 23
   <212> DNA
   <213> Artificial sequence
<220> primer_bind
   <223> RhoR primer for amplification of rho gene
<400> 2
   ttatgagcgt ttcatcattt cga
   23
<210> 3
   <211> 19
   <212> DNA
   <213> Artificial sequence
<220> primer_bind
   <223> MlambdaRf primer for amplification prophage lambda c1857
<400> 3
   gtatgcatgc tgggtgtgg
   19
<210> 4
   <211> 25
   <212> DNA
   <213> Artificial sequence
<220> primer_bind
   <223> MlambdaRr primer for amplification prophage lambda c1857
<400> 4
   cgcactctcg attcgtagag cctcg
   25
<210> 5
   <211> 24
   <212> DNA
   <213> Artificial sequence
<220> primer_bind
   <223> MobAF primer for amplification of T4 mobA gene
<400> 5
   gtagaaaata gtgctaaaaa gtgt
   24
<210> 6
   <211> 22
   <212> DNA
   <213> Artificial sequence
<220> primer_bind
   <223> MobAR primer for amplification of T4 mobA gene
<400> 6
   ttaatagtgc ggggtaaaac cc
   22